(19) Europäisches Patentamt / European Patent Office / Office européen des brevets

(11) **EP 3 453 769 B1**

(12) **EUROPEAN PATENT SPECIFICATION**

(45) Date of publication and mention of the grant of the patent:
**26.04.2023 Bulletin 2023/17**

(21) Application number: **18194115.4**

(22) Date of filing: **12.09.2018**

(51) International Patent Classification (IPC):
*C12Q 1/6858* (2018.01)    *C12Q 1/686* (2018.01)

(52) Cooperative Patent Classification (CPC):
(C-Sets available)
**C12Q 1/6858; C12Q 1/686**    (Cont.)

(54) **MULTIPLEX NUCLEIC ACID DETECTION METHOD**

MULTIPLEX-NUKLEINSÄURENACHWEISVERFAHREN

PROCÉDÉ DE DÉTECTION D'ACIDES NUCLÉIQUES MULTIPLEX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.09.2017 CN 201710815042**

(43) Date of publication of application:
**13.03.2019 Bulletin 2019/11**

(73) Proprietor: **LifeOS Genomics Corporation**
**1208 Grand Cayman (KY)**

(72) Inventor: **LIU, Timothy Z**
**Fremont, CA 94555 (US)**

(74) Representative: **Boult Wade Tennant LLP**
**Salisbury Square House**
**8 Salisbury Square**
**London EC4Y 8AP (GB)**

(56) References cited:
**WO-A1-2010/115100    WO-A1-2012/129436**
**US-A1- 2008 254 474    US-A1- 2014 378 320**

- **CAO LEI ET AL: "Advances in digital polymerase chain reaction (dPCR) and its emerging biomedical applications", BIOSENSORS AND BIOELECTRONICS, ELSEVIER SCIENCE LTD. UK, AMSTERDAM, NL, vol. 90, 25 September 2016 (2016-09-25), pages 459-474, XP029862188, ISSN: 0956-5663, DOI: 10.1016/J.BIOS.2016.09.082**

- **JASON E KREUTZ ET AL: "Theoretical design and analysis of multivolume digital assays with wide dynamic range validated experimentally with microfluidic digital PCR", ANALYTICAL CHEMISTRY, AMERICAN CHEMICAL SOCIETY, US , vol. 83, no. 21 1 November 2011 (2011-11-01), pages 8158-8168, XP002693075, ISSN: 0003-2700, DOI: 10.1021/JA2060116 Retrieved from the Internet: URL:http://pubs.acs.org/doi/abs/10.1021/ja 2060116**

- **FENG SHEN ET AL: "Multiplexed Quantification of Nucleic Acids with Large Dynamic Range Using Multivolume Digital RT-PCR on a Rotational SlipChip Tested with HIV and Hepatitis C Viral Load", JOURNAL OF THE AMERICAN CHEMICAL SOCIETY, vol. 133, no. 44, 9 November 2011 (2011-11-09), pages 17705-17712, XP055054910, ISSN: 0002-7863, DOI: 10.1021/ja2060116**

EP 3 453 769 B1

(52) Cooperative Patent Classification (CPC): (Cont.)

C-Sets
**C12Q 1/6858, C12Q 2525/155, C12Q 2527/146,
C12Q 2537/143, C12Q 2563/159, C12Q 2563/179,
C12Q 2565/537;
C12Q 1/686, C12Q 2525/155, C12Q 2527/146,
C12Q 2537/143, C12Q 2563/159, C12Q 2563/179,
C12Q 2565/537**

**Description**

**BACKGROUND**

Field of Invention

The present invention disclosure relates to methods for PCR, and more particularly to methods for digital PCR for detecting multiplex nucleic acid segments of interest.

Description of Related Art

Polymerase chain reaction (PCR) has been widely applied in many fields of nucleic acid analysis. Digital PCR (also known as single molecule based PCR) is a relatively new development in the field of PCR technology. Compared to the conventional ways, the significant difference in quantification between digital PCR and convention quantitative PCT is that the sample to be tested is separated into a large number of small portions for amplification reaction, and there is less than one template molecule on average for each of the small portions. Some of the small portions have no template molecule, some have more than one template molecule, and a certain percentage of the small portions have just one template molecule. After the amplification reaction, the copy number of the template molecules in the sample is determined by detecting the presence or absence of the template molecule in the respective small portions. As to whether the sample has a specific target sequence (i.e Target segments), the analysis results provide "yes" or "no" binary signals, e.g., in a digital format 0 or 1. Through statistical analysis the target nucleic acid segment can be quantified by the results of all small separations in the digital PCR. Digital PCR can be used to directly calculate the copy number of the target nucleic acid segment, so absolute quantification can be carried out without reference samples and standard curve.

Now digital PCR has been widely applied for the detection of gene sequence variations, for example, copy number variants or point mutations. In molecular diagnosis, digital PCR is applied in cancer, infectious disease, fetal genetic screening and prediction of transplant rejection, microRNA research, assessment of allelic imbalance, quantitative analysis of gene expression, DNA methylation status analysis, pathogenic microorganism detection, etc.

Many diseases are caused by "a combination of multiple genetic mutations" plus environmental factors. Taking hypertension as an example, it has been inferred that more than 300 genetic mutations are related to the risk of hypertension. However, it is still inconclusive as to how many genetic mutations in combination plus environmental factors will make the incidence of hypertension. Nevertheless, the detection of risk loci in related diseases is of great benefit to early detection and prevention of diseases.

However, the current digital PCR method is performed by monitoring the amplification reactions in each small separation through the addition of fluorescent dyes, or fluorescence-labeled probes into PCR reaction systems, so that the number of target nucleic acid segments that can be simultaneously detected is limited by the species of fluorescent marker and the optical resolution of available fluorescent markers. Therefore, there is a need to further improve the current digital PCR method to enhance the capability of multiplex quantitative analysis of target molecules. WO 2010/115100 A1 discloses multiplex nucleic acid detection methods and systems.

**SUMMARY**

Some embodiments of the present disclosure provide a method for analyzing multiplex target nucleic acid segment in a sample, the sample may contain a first target nucleic acid segment and a second target nucleic acid segment. The method comprises the following steps: (a) performing a pre-amplification to generate first template molecules from the first target nucleic acid segment and generate second template molecules from the second target nucleic acid segment, wherein each of the first template molecules comprises sequences generated from a first universal primer sequence, a second universal sequence, and a first identity sequence tag, and each of the second template molecules comprises sequences generated from the first universal primer sequence, the second universal sequence, and a second identity sequence tag; (b) dispersing the sample to a substrate in a flow cell for random distribution of the first template molecules and the second template molecules among respective reaction wells of the substrate, wherein the number of the first template molecules and the second template molecules in each of the reaction wells is less than 1 on average; (c) in situ performing nucleic acid amplification reaction to generate a plurality of first amplicons from the first template molecules and a plurality of second amplicons from the second template molecules, wherein the first amplicons and the second amplicons are fixed on the respective inner surfaces within the respective reaction wells of the substrate in the flow cell; (d) in situ identifying the first identity sequence tag of the first template molecules of the first amplicons and the second identity tag of the second template molecules of the second amplicons on the respective inner surfaces within the respective reaction wells of the substrate in the flow cell, wherein a signal of the first identity sequence tag represents a positive identification of the first target nucleic acid segment, and a signal of the second identity sequence tag represents a positive identification of the second target nucleic acid segment; and (e) performing statistical calculation for quantifying the first target nucleic acid segment and the second target nucleic acid segment through the number of the reaction wells, the number of the positive identification of the first target nucleic acid segment, and the number of the positive identification of the second target nucleic acid segments.

According to one embodiment, wherein the first identity sequence tag comprises a first identity code sequence, the second identity sequence tag comprises a second identity code sequence.

According to one embodiment, wherein the pre-amplification reaction comprises gene-specific amplification.

According to one embodiment, wherein the pre-amplification reaction comprises gene-specific 5' exonuclease cleavage reaction.

According to one embodiment, wherein the pre-amplification reaction comprises a linkage reaction of target-specific probes and then a universal primer sequence based amplification reaction, and the target-specific probes comprise the first identity sequence tag or the second identity sequence tag.

According to one embodiment, wherein the sample further contains a reference nucleic acid segment, and each of template molecules generated from the reference nucleic acid segment has a third identity sequence tag.

According to one embodiment, wherein each of the first template molecules further comprises a sequence generated from the first target nucleic acid segment, and each of the second template molecules further comprises a sequence generated from the second target nucleic acid segment.

According to one embodiment, wherein the reaction wells of the substrate are recessed spaces formed on the surface of the substrate.

According to one embodiment, the reaction wells of the substrate are through holes formed through the substrate.

According to one embodiment, the reaction wells of the substrate have the same volume.

According to one embodiment, wherein the reaction wells of the substrate are circular or polygonal in shape.

According to one embodiment, wherein the reaction wells of the substrate have different shapes.

According to one embodiment, the method further includes: after the pre-amplification reaction of the step (a), the nucleic acid amount of the first template molecules or the second template molecules are quantified, and the dilution factor of the sample is determined.

According to one embodiment, the method further comprises: before the step (c), a water-immiscible liquid is added to the substrate to cover and isolate the reaction wells from each other.

According to one embodiment, wherein third primer sequences are fixed within the reaction wells, each of the third primer sequences comprises a sequence complementary or identical to the first universal primer sequence or the second universal primer sequence.

According to one embodiment, wherein the first amplicons and the second amplicons are attached to the inner surfaces of the reaction wells through the third primer sequences.

According to one embodiment, wherein the first amplicons generated from the first template molecules and the second amplicons generated from the second template molecules are immobilized on the inner surfaces of the reaction wells through an intermediate matrix.

According to one embodiment, DNA sequencing, polymerase extension, probe hybridization, or probe ligation is used to identify the identity sequence tags.

According to one embodiment, the method further comprises analyzing the sequence composition other than the first identity sequence tag of the first amplicons, or the sequence composition other than the second identity sequence tag of the second amplicons.

According to one embodiment, the first target nucleic acid segment and the second target nucleic acid segment are the same nucleic acid segment.

According to one embodiment, the first target nucleic acid segment and the second target nucleic acid segment are different nucleic acid segments.

According to one embodiment, step (b) of the method further comprises: the reaction solution flows over the respective inner surfaces of the reaction wells of the substrate.

The sensitivity and accuracy of digital PCR for the multiplex quantitative analysis of target molecules can be enhanced by generating respective template molecules having specific identity sequence tags, and performing *in situ* amplification and identification of the respective identity sequence tags in the separated reaction wells.


## BRIEF DESCRIPTION OF THE DRAWINGS

The invention can be more fully understood by reading the following detailed description of the embodiment, with reference made to the accompanying drawings as follows:

Fig. 1 illustrates a flow chart of a nucleic acid detection method in accordance with some embodiments of the present disclosure;

Fig. 2A illustrates a schematic diagram of the steps of a pre-amplification reaction in accordance with some embodiments of the present disclosure;

Fig. 2B illustrates a schematic diagram of a pre-amplification reaction in accordance with some embodiments of the present disclosure;

Fig. 3A illustrates a schematic top view of a substrate in accordance with some embodiments of the present disclosure;

Fig. 3B illustrates a schematic top view of a substrate in accordance with some embodiments of the present disclosure;

Fig. 3C shows a top view image of a substrate in accordance with one example of the present disclosure;

Fig. 3D shows a side view of a substrate in accordance with one embodiment of the present disclosure;

Fig. 4A illustrates a schematic diagram of the clonal amplification reaction in accordance with some embodiments of the present disclosure;

Fig. 4B illustrates a schematic diagram of an amplification reaction in accordance with some embodiments of the present disclosure;

Fig. 4C illustrates a schematic diagram of an amplification reaction in accordance with some embodiments of the present disclosure;

Fig. 4D illustrates a schematic diagram of an amplification reaction in accordance with some embodiments of the present disclosure;

Fig. 5 illustrates a schematic diagram of label types and base compositions in accordance with some embodiments of the present disclosure;

Fig. 6 illustrates a process for identifying identity codes in accordance with some embodiments of the present disclosure;

Fig. 7 shows a fluorescent image in accordance with one example of the present disclosure;

Fig. 8A illustrates a perspective view of a flow cell in accordance with some embodiments of the present disclosure;

Fig. 8B illustrates a perspective view of a flow cell and shows the reaction liquid can flow over the substrate having the reaction wells in accordance with some embodiments of the present disclosure;

Fig. 9 illustrates a side schematic view of a nucleic acid detection system in accordance with some embodiments of the present disclosure.

**DETAILED DESCRIPTION**

Reference will now be made in detail to the present embodiments of the invention, examples of which are illustrated in the accompanying drawings. Wherever possible, the same reference numbers are used in the drawings and the description to refer to the same or like parts.

Some embodiments of the present disclosure provide the methods and systems for simultaneous and multiplex quantitative analysis of target molecules (e.g., Target nucleic acid segments), such as detection of multiple biomarkers for discovery, validation, or clinical diagnosis.

As used herein, "target nucleic acid segment," "target segment" or a grammatical equivalent means a nucleic acid segment or its complementary segment on a single strand of nucleic acid. A target nucleic acid segment may be a part of a gene, a regulatory sequence, genomic DNA, cDNA, RNA (e.g., mRNA and rRNA) or a part of another nucleic acid segment. A target nucleic acid segment may be any length, with the understanding that longer segments are more specific. In some embodiments, it may be desirable to fragment or cleave the sample nucleic acid into fragments of 100 to 10,000 base pairs, with fragments of roughly 500 base pairs being preferred in some embodiments. As will be appreciated by those in the art, the complementary target segment may take many forms. For example, it may be contained within a larger nucleic acid segment, i.e. all or a part of a gene or mRNA, a restriction fragment of a plasmid or genomic DNA, among others.

In some embodiments, the different target nucleic acid segments are within the same gene. In some embodiments, the different target nucleic acid segments are within different genes. Some embodiment of the present disclosure may be used to detect single nucleotide polymorphisms (SNP).

In some embodiment, the first target nucleic acid segment and the second target nucleic acid segment are the same nucleic segment. Therefore, the method provided in the present disclosure can be applied to quantify a single target nucleic acid segment.

In some embodiment, the first target nucleic acid segment and the second target nucleic acid segment are different segments, such that the method provided in the present disclosure can be used to simultaneously quantify multiple different target nucleic acid segments.

In some embodiment of the present disclosure, each of the target nucleic acid segments is assigned a respective identity sequence tag in an encoded manner; then the respective target sequence tags can represent the corresponding target nucleic acid segments.

As described herein, "an identity sequence tag" is used to represent a short artificial DNA sequence. In some embodiment, an identity sequence tag includes an identity code sequence of a specific target nucleic acid segment. In some embodiment, the length of an identity code sequence is shorter than 6 bases or shorter than 10 bases. In some embodiments, the length of an identity code sequence may be from 4 bases to 8 bases. In some embodiments, the length of an identity code sequence may be from 10 bases to 20 bases. The identity sequence tag may also contain additional nucleic acid sequences, which are required for hybridization of probes during a decoding process. Typically, the identity code sequence is designed to be different from the sequence of the genome of interest in the nucleic acid analysis. In some embodiment of the disclosure, introducing an identity sequence tag comprising an identity code sequence is a part of sample preparation.

However, not all possible base sequence combinations are suitable for being identity code sequences due to self-pairing or interacting with nucleic acid molecules. For example, using a palindromic sequence (e.g., ACGT) may not be a good choice as an identity code sequence. Therefore, the number of available identity code sequences may be less than the theoretical numbers.

As described herein, the information of an identity code sequence can be converted to "identity code". "Identity code" is applied to indicate the code assigned to the identity sequence tag. Each of the identity sequence tags has a specific identity code sequence.

Some embodiments of the present disclosure provide methods of genome-specific or gene-specific pre-amplification that incorporates a specific identity sequence tag having an identity code sequence in each of the generated template molecules generated from the target nucleic acid segment.

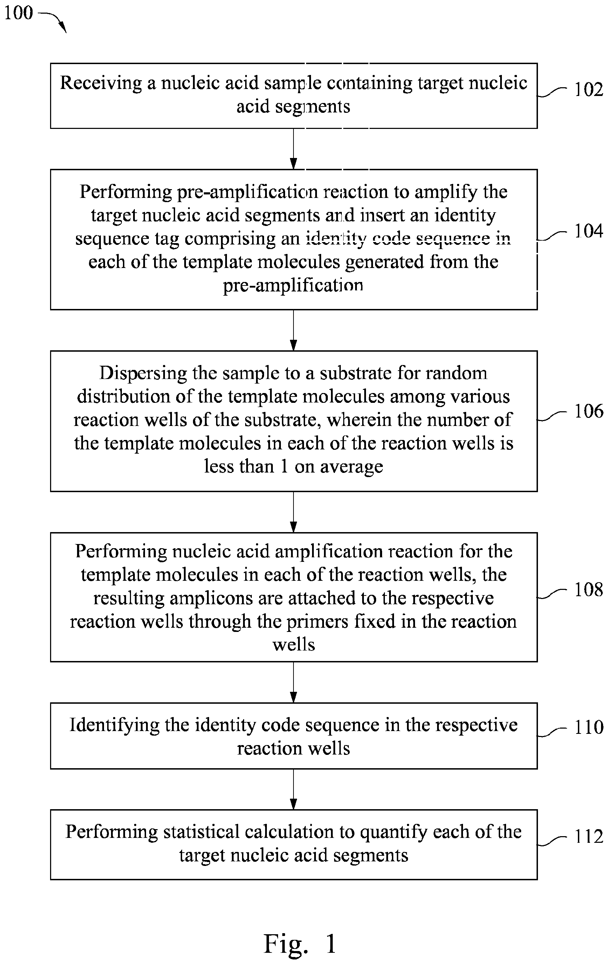

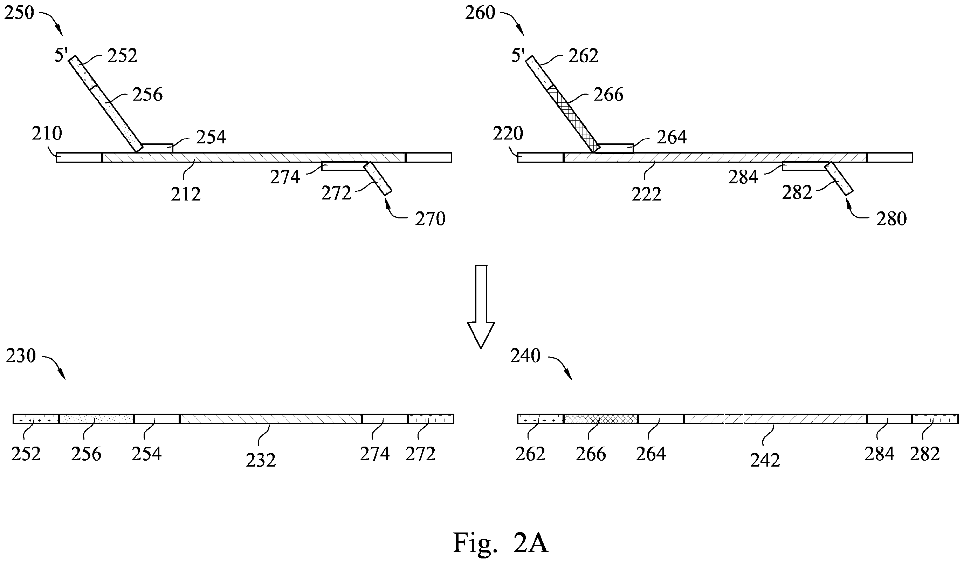

Fig. 1 shows a flow chart of the method 100 for detection of nucleic acid, according to various embodiments of the present disclosure. Some steps of the method 100 will be described in detail later with reference to Figs. 2A, 2B, 3A, 3B, 3C, 3D, 4A, 4B, 5, and 6.

Please refer to Fig. 1. In step 102 of the method 100, a sample containing nucleic acid (i.e., A nucleic acid sample) is received, the sample may contain target nucleic acid segments of interest.

In some embodiments, some target nucleic acid segments of interest may not exist in the samples, that is, the quantitative results will show that the numbers of these target nucleic acid segments are zero.

In some embodiments, the target nucleic acid segments comprise an endogenous reference sequence, e.g., a segment of a housekeeping gene.

In some embodiments, an exogenous nucleic acid segment is added to the sample as a target nucleic acid segment, and the copy number of the exogenous nucleic acid segment is predetermined.

As used herein, "nucleic acid" means deoxyribonucleic acid (DNA) or ribonucleic acid (RNA) and its single chain, double chain, or any chemical modifications thereof (e.g., Methylation, ethylation, etc.).

The sample comprising the target segment may be from virtually any organism, and any sources, including, but not limited to, bodily fluids (including, but not limited to, blood, bone marrow, urine, feces, tears, serum, lymph, saliva, anal and vaginal secretions, perspiration, semen, and other bodily fluids of virtually any organism, such as mammalian, including human, samples); cell lysates of bacteria and pathogens, including viruses; hard tissues (e.g., organs such as liver, spleen, kidney, heart, lung, etc.); environmental samples (including, but not limited to, air, agricultural, water and soil samples); biological warfare agent samples; research samples (i.e., The sample may be the products of an amplification reaction); purified samples, such as purified genomic DNA, RNA, proteins, etc.; raw samples (bacteria, virus, genomic DNA, etc.).

A. Pre-amplification reaction

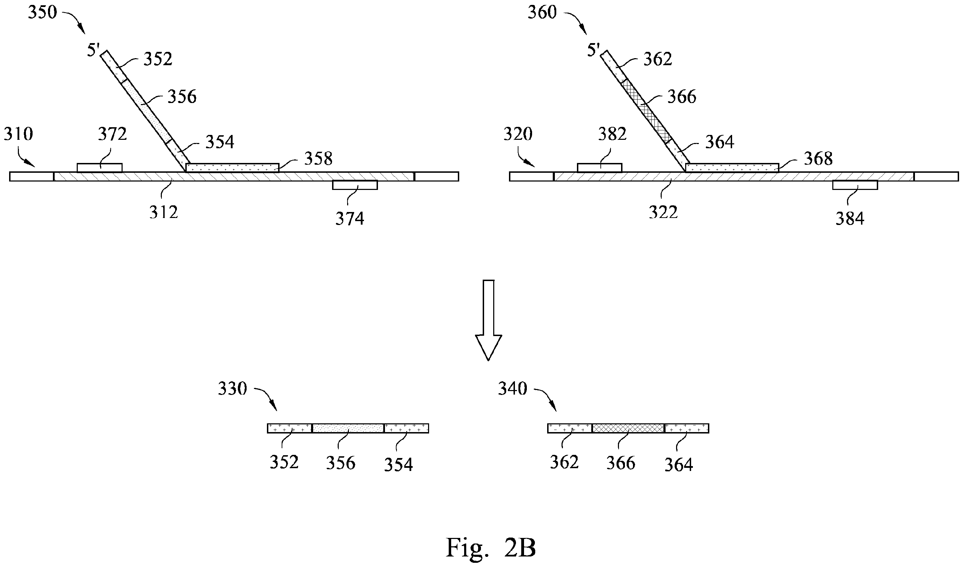

Please refer to Fig. 1, 2A, and 2B. After receiving the nucleic acid sample, in step 104 of the method 100, a pre-amplification reaction is performed to amplify the target nucleic acid segments and insert an identity sequence tag comprising an identity code sequence in each of the template molecules generated in the pre-amplification reaction.

Please refer to Fig. 2A, which shows a schematic diagram of a gene-specific pre-amplification reaction according to one embodiment. The nucleic acid sample comprises a first target nucleic acid molecule 210 having a first target nucleic acid segment 212, and a second target nucleic acid molecule 220 having a second target nucleic acid segment 222. During pre-amplification reaction, a primer 250 and a primer 270 are used as primers for pre-amplification reaction, the sequence of the first target nucleic acid segment 212 is used as a template for pre-amplification to generate the first template molecule 230. Correspondingly, a primer 260 and a primer 280 are used as primers for pre-amplification reaction, the sequence of the second target nucleic acid segment 222 is used as a template for pre-amplification to generate the second template molecule 240.

Please still refer to Fig. 2A. The primer 250 comprises a first universal primer sequence 252, a first identity sequence tag 256, and a second primer sequence 254 complementary to a partial segment of the first target nucleic acid segment 212. The primer 260 comprises a first universal primer sequence 262, a second identity sequence tag 266, and a second primer sequence 264 complementary to a partial segment of the second target nucleic acid segment 222. Wherein the first universal primer sequence 252 and the first universal primer sequence 262 are the same sequences.

The primer 270 comprises a second universal primer sequence 272, and a specific primer sequence 274 complementary to a partial segment of the first target nucleic acid segment 212. The primer 280 comprises a second universal primer sequence 282, and a specific primer sequence 284 complementary to a partial segment of the second target nucleic acid segment 222. Wherein the second universal primer sequence 272 and the second universal primer sequence 282 are the same sequence.

In one embodiment, the first universal primer sequence and the second universal primer sequence may be different sequences.

The first identity sequence tag 256 and the second sequence identity sequence tag 266 respectively have different identity code sequences. The first identity sequence tag 256 comprises a first identity code sequence, and the second identity sequence tag 266 comprises a second identity code sequence (not shown).

Please still refer to Fig. 2A. After the pre-amplification reaction, the resulting first template molecule 230 comprises a first target template sequence 232, and sequences generated from the first universal primer sequence 252, the second universal primer sequence 272 (respectively at each end of the first template molecule 230), and the first identity sequence tag 256 (i.e., It is inserted into the first template molecule 230). Correspondingly, the resulting second template molecule 240 comprises a second target template sequence 242, and sequences generated from the first universal primer sequence 262, the second universal primer sequence 282 (respectively at each end of the second template molecule 240), and the second identity sequence tag 266 (i.e., It is inserted into the second template molecule 240). Therefore, the 5' ends of the first template molecule 230 and the second template molecule 240 have the same universal primer sequence, and the 3' ends of the first template molecule 230 and the second template molecule 240 have another the same universal primer sequence.

In one embodiment, a relatively small number of PCR cycles can be used to carry out gene-specific pre-amplification, preferably less than 30 cycles, 20 cycles, or 10 cycles, to provide sufficient template molecules and preserve the ratios among different target nucleic acid segments in the original sample.

Please refer to Fig. 2B, which illustrates a method of a gene-specific 5' exonuclease cleavage pre-amplification reaction for inserting an identity sequence tag into a template molecule. The polymerase of this reaction has 5' to 3' exonuclease activity, such as Tag polymerase. The nucleic acid sample comprises a first target nucleic acid molecule 310 having a first target nucleic acid segment 312, and a second target nucleic acid molecule 320 having a second target nucleic acid segment 322.

Probes are designed for the first target nucleic acid segment 312 and the second target nucleic acid segment 322, respectively, and the probes function similarly to the probes of TaqMan real-time PCR, in which the 3' flap of the probe is complementary to the target sequence, and the 5' flap of the probe is cut from the probe by the 5' exonuclease during polymerase amplification.

The 5' flap of the probe 350 comprises a first universal primer sequence 352, a first identity sequence tag 356, and a second universal primer sequence 354. The 3' flap of the probe 350 comprises a gene-specific probe sequence 358 complementary to a partial segment of the first target nucleic acid segment 312. The 5' flap of the probe 360 comprises a first universal primer sequence 362, a second identity sequence tag 366, and a second universal primer sequence 364. The 3' flap of the probe 360 comprises a gene-specific probe sequence 368 complementary to a partial segment of the second target nucleic acid segment 322. Wherein the first universal primer sequence 352 and the second universal primer sequence 362 are the same universal primer sequence, and the second universal primer sequence 354 and the second universal primer sequence 364 are the same universal primer sequence.

In one embodiment, the first universal primer sequence and the second universal primer sequence may be different sequences.

Please still refer to Fig. 2B. Primer 372 and primer 374 are respectively complementary to a partial sequence near each of the ends of the first target nucleic acid segment 312. Primer 382 and primer 384 are respectively complementary to a partial sequence near each of the ends of the second target nucleic acid segment 322.

After the probes hybridizing with the target nucleic acid segments, during the polymerase reaction, the first template molecule 330 is cut from the probe 350, and the second template molecule 340 is cut from the probe 360 because of the activity of the 5' exonuclease.

The first template 330 comprises sequences generated from the first universal primer sequence 352, the first identity sequence tag 356, and the second universal primer sequence 354. The second template 340 comprises sequences generated from the first universal primer sequence 362, the first identity sequence tag 366, and the second universal primer sequence 364.

Because the probes used in the 5' exonuclease cleavage pre-amplification are specifically designed for the respective specific target nucleic acid segments of interest, the first template 330 becomes a surrogate of the first target nucleic acid molecule 310, and the second template 340 becomes a surrogate of the second target nucleic acid molecule 320.

In one embodiment, the pre-amplification reaction of the target nucleic acids may comprise a ligation reaction of target-specific probes containing an identity sequence tag, and followed by an amplification reaction with universal primer sequences, thereby multiple template molecules respectively with identity sequence tags are simultaneously generated. Such method of pre-amplification reaction to generate template molecules has been published in the literature, e.g. Castiglionic Appl. Environ. Microbiol., 2004, 70 (12), 7161, which is cited in the disclosure.

In one embodiment, the genetic DNA of an organism is treated with sodium bisulfite for DNA methylation analysis. Methylation-specific primers and probes can be used in the 5' exonuclease cleavage pre-amplification of the target segments. According to one embodiment, the method may be applied to detect whether the sequences of the target nucleic acid segment in an original sample are methylated.

In some embodiments, the nucleic acid in the sample is ribonucleic acid (RNA), for example, in the case of gene expression analysis. Prior to carrying out the pre-amplification, a reverse-transcription reaction is performed to reverse transcript the RNA into cDNA.

In some embodiment, the first and the second target nucleic acid segments are similar in abundance. In some embodiments, the first target nucleic acid segment is at least 100, 1000, or 10,000 times more abundant than the second target nucleic acid segment.

B. Dispersing the sample randomly to each of the reaction wells

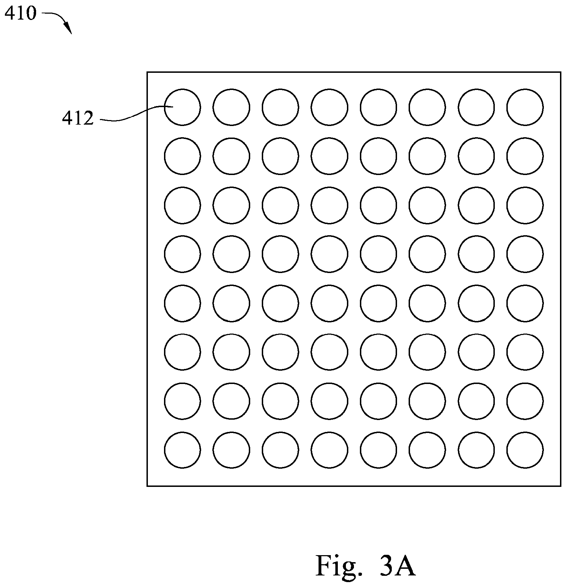

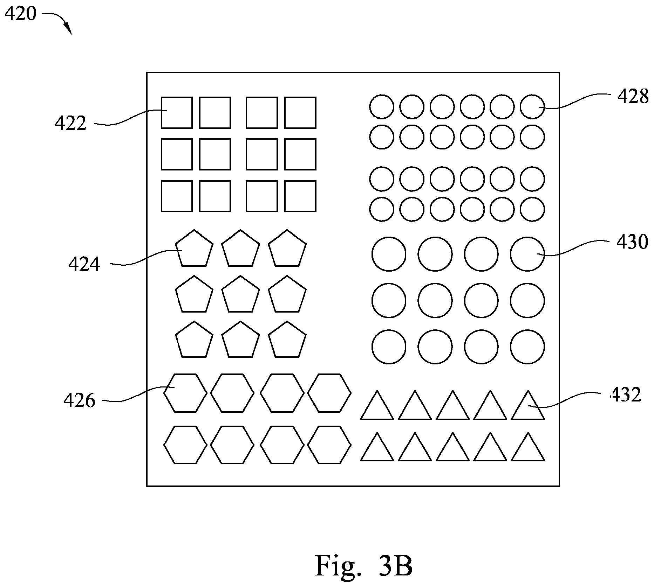

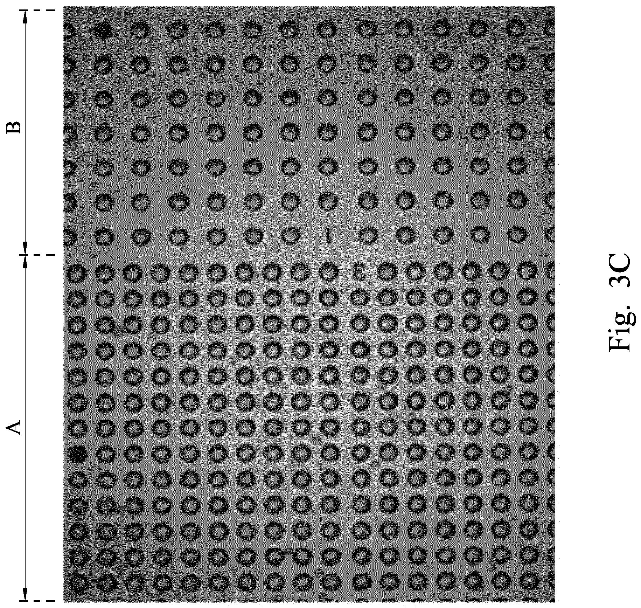

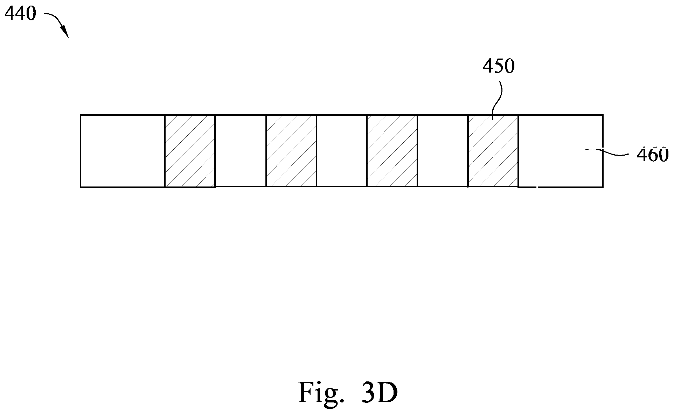

Please refer to Fig. 1, 3A, 3B, 3C, and 3D. In step 106 of the method 100, the sample comprising the template molecules is randomly dispersed into the various reaction wells, such that the number of the first template molecule or the second template in each of the reaction wells is less than 1 on average.

According to some embodiments, recessed spaces are formed on the substrate by microelectronics fabrication methods, such as photolithography, to generate a substrate having a plurality of physically separated micropores, which are the reaction wells for performing the amplification reaction. For example, a uniform photoresist layer is coated on a polished surface of a substrate by spin coating, and then the micropores are formed by etching (e.g., Reactive ion etching). The material of the substrate may be or comprise silicon, glass, plastic, or metal (e.g., Aluminum, or stainless steel). According to some embodiments, the material of the substrate has a low fluorescent background value; therefore, the material is compatible with fluorescence detection. According to some embodiments, the inner surfaces of the reaction wells are compatible with the enzymatic reactions.

In some embodiments, the surface of the reaction wells can be treated with a surface coating, such as a hydrophilic surface coating.

In some embodiments, the substrate has at least 5,000 wells, 10,000 wells, or 30, 000 wells. In some embodiments, the substrate has less than 200,000 wells, 100,000 wells, 50,000 wells, or 10,000 wells.

In some embodiments, since the number of the reaction wells in a substrate is predetermined, the volume of the sample during a reaction process can be obtained through the number and volume of the reaction wells.

According to some embodiments, the side length or diameter of reaction wells may be in a range from 2 $\mu$m to 1000 $\mu$m, preferably from 10 $\mu$m to 500 $\mu$m, more preferably from 50 $\mu$m to 200 $\mu$m. The depth of reaction wells may be in a range from 10 $\mu$m to 1,000 $\mu$m, preferably from 10 $\mu$m to 500 $\mu$m, more preferably from 20 $\mu$m to 100 $\mu$m.

According to some embodiments, various reaction wells may be arranged in various forms or patterns on a substrate.

Please refer to Fig. 3A, which shows a schematic top view of a substrate 410 according to some embodiments. The substrate 410 has a plurality of reaction wells 412 with the same size and same pitch.

Please refer to Fig. 3B, which shows a schematic top view of a substrate 420 according to some embodiments. The substrate 420 has reactions wells with different shapes. The reaction well 422 is a rectangular shape; the reaction wells 424 is a pentagonal shape; the reaction well 426 is a hexagonal shape; the reaction well 430 is a circular shape; the reaction well 430 is another circular shape with a diameter greater than that of the reaction well 428; the reaction well 432 is a triangular shape.

Please refer to 3C, which shows a top view image of a substrate according to one example. The diameters of the various circular reaction wells are the same, for example, the diameter of each of the reaction wells is 80 $\mu$m, and the depth is 50 $\mu$m to 100 $\mu$m. The distance between the centers of the adjacent reaction wells in region A is 100 $\mu$m; the distance between the centers of the adjacent reaction wells in region B is 120 $\mu$m.

Please refer to Fig. 3D, which shows a cross-sectional view of a substrate according to one example, wherein the reaction wells are through holes through the substrate. Fig. 3D illustrates the substrate 440, and the reaction wells 450 are through holes through the substrate 460.

When the aqueous sample solution containing the reaction reagents and the template molecules is passed over the substrate, the aqueous sample solution can flow into each of the reaction wells of the substrate. Since the reactions are physically separated from each other, the reactions in the respective reaction wells are independent of each other. Therefore, the aqueous sample can be effectively allocated to a large number of respective separated reaction spaces. Such sample separation method is faster and more economical, compared to the dispensing method using the latex droplet system.

In some embodiments, the sample can be divided into repeats (e.g., Duplicate, triplicate, etc.) for measuring the amount of the target nucleic acid segment. The sample can be derived from the same source, and divided into repeats before the amplification reaction. Additionally, serial dilutions of the sample can be generated. Repeated and diluted samples can be analyzed in a continuous or parallel manner.

Each of the reaction wells comprises about 1 or less template molecule on average and reagents sufficient for performing the polymerase reaction.

Compared to prior art "droplet in oil" digital PCR, embodiments of the present disclosure significantly simplify the step of the sample separation without emulsification of the reaction solution with an oil phase solution; therefore, the separation step for the reaction liquid is more cost-effective, more efficient, and faster.

In some embodiments, a sealing liquid can be added to quickly cover the surface of the substrate for preventing evaporation during thermal cycling of the PCR reaction and isolating each of the reaction wells. The sealing liquid is immiscible with water, compatible with the enzymatic reaction, and can be easily removed from the surface after thermal cycling. According to some embodiments, the preferred sealing liquid comprises mineral oil. If the reaction well is a through hole, the sealing liquid should cover both ends of the through hole during thermal cycling of the PCR.

According to some embodiments, the template molecules are dispersed into a large number of individual reaction wells. In the subsequent steps, there is less than one copy of the template molecules on average in each of the reaction wells. Dilution of template molecules might be needed to achieve the goal of less than one copy of the template molecule in each PCR.

According to some examples, most of the individual reaction wells contain only single template wells.

C. Clonal amplification reaction

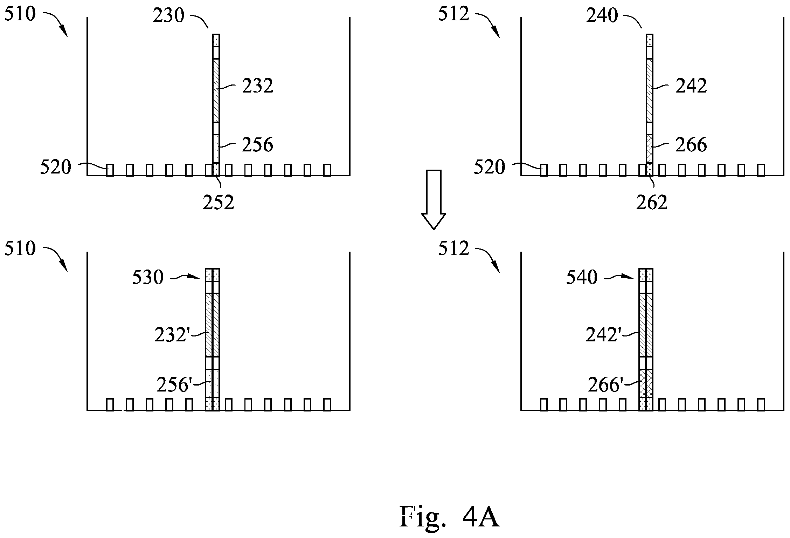

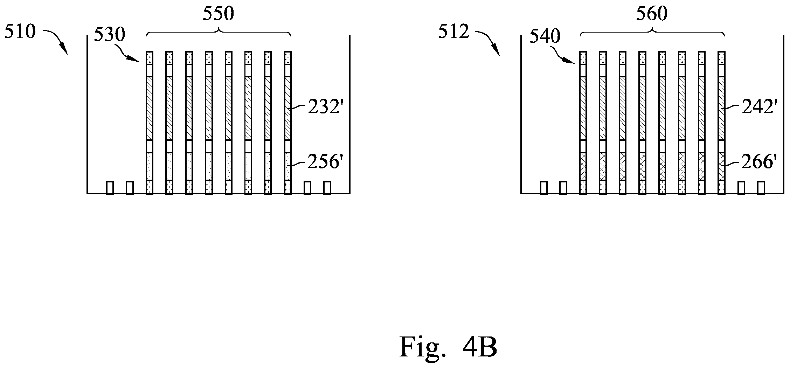

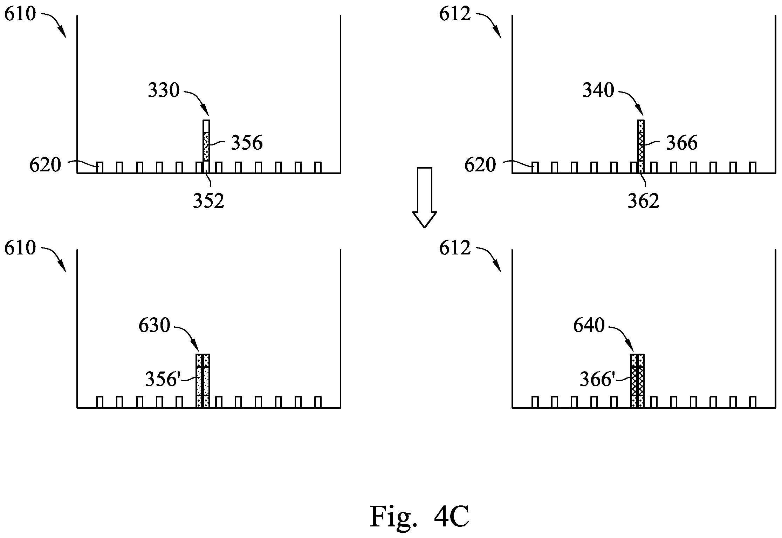

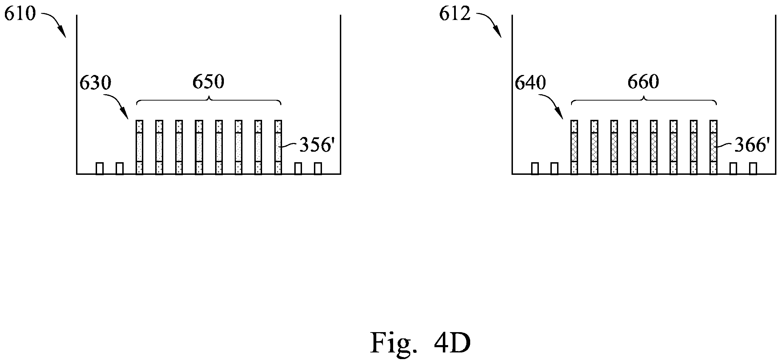

Please refer to Fig. 1, 4A, 4B, 4C, and 4D. In step 108 of the method 100, a nucleic acid amplification reaction is performed in situ for the template molecules in each of the reaction wells, the resulting amplicons are attached to the reaction wells through the primers fixed in the reaction wells.

According to some embodiments, the primer sequences complementary to the universal primer sequence of the template molecules generated from the pre-amplification reaction are fixed in the respective inner surfaces of the reaction wells.

According to some embodiments, the inner surface of the reaction well may have primers complementary to the specific primer sequences of the template molecules generated from the pre-amplification reaction.

According to some embodiments, the primers are immobilized on the respective surfaces of the reaction wells by a physical force or by a chemical linkage.

According to some embodiments, each of the primers is coupled to the inner surface of a reaction well by a disulfide bond.

Attachment of the primer to the inner surface of the reaction well can be accomplished through various surface chemistries known in the art.

According to some embodiment of the present disclosure, the primers may be coupled to an intermediate matrix, for example, magnetic nanoparticles. The materials for the magnetic nanoparticles may be or comprises $Fe_2O_3$, $Fe_3O_4$, $COFe_2O_4$ or $MnFe_2O_4$. The sizes of the magnetic nanoparticles may be in a range from 10 nm to 500 nm, preferably from 15 nm to 100 nm; more preferably from 15 nm to 50 nm. Magnetic nanoparticles having primer sequences are added to the reaction wells of the substrate, and a magnetic field is applied under the substrate to confine the magnetic nanoparticles having the primers to the bottom of the reaction wells. Magnetic fields can be applied through various technologies known in this field, such as through a permanent magnet or an electromagnetic mechanism.

Please refer to step 108 of Fig. 1 and Fig. 4A. A first template molecule 230 generated from the gene-specific pre-amplification reaction is within a first reaction well 510, and a second template molecule 240 is within a second reaction well 512. The third primer sequences 520 are immobilized on the inner surface of the first reaction well 510 and the second reaction well 512, and the sequence of the third primer sequence 520 is identical or complementary to the first universal primer sequence 252, 262, or the second universal primer sequence 272, 282 as shown in Fig. 2A. The third primer sequence 520 is used as a primer for the step of a clonal amplification reaction. In the first reaction well 510, the first template molecule 230 is used as a template, and the first amplicon 530 generated is fixed in the first reaction well 510. The first amplicon 530 has a sequence 232' complementary to the first target template sequence 232, and sequence 256' complementary to the first identity sequence tag 256. Correspondingly, in the second reaction well 512, the second template molecule 240 is used as a template, and the second amplicon 540 generated is fixed in the first reaction well 512. The second amplicon 540 has a sequence 242' complementary to the second target template sequence 242, and sequence 266' complementary to the second identity sequence tag 266.

Then, when the thermal cycling reaction is heating up, the first template molecule 230 is separated from the first amplicon 530, and the second template molecule 240 is separated from the second amplicon 540. Then, when the thermal cycling reaction is cooling down, because the reaction solution contains the primer sequences identical or complementary to the first universal primer sequences 252, 262, and primer sequences identical or complementary to the second universal primer sequences 272, 282, a segment identical to the first template molecule 230 is generated by using the first amplicon 230 as a template, and a segment identical to the second template molecules 240 is generated by using the second amplicon 240 as a template. During the subsequent thermal cycles, in the first reaction well 510, the first amplicons 530 fixed in the reaction well are generated through using the third primer sequences 520 fixed in the reaction well as primers, and using the segments identical to the first template molecule 230 as templates. In the second reaction well 512, the second amplicons 540 fixed in the reaction well are generated through using the third primer sequences 520 fixed in the reaction well as primers, and using the segments identical to the first template molecule 240 as templates.

Please refer to Fig. 4B, during the clonal amplification reaction, in the first reaction well 510, many first amplicons 530 fixed in the reaction well are generated and a first amplicon cluster 550 is formed. In the second reaction well 512, many second amplicons 540 fixed in the reaction well are generated and a second amplicon cluster 560 is formed.

Please refer to step 108 of Fig. 1 and Fig. 4C. A first template 330 generated in the gene-specific 5' exonuclease cleavage pre-amplification reaction is in the first reaction well 610, and a second template molecule 340 is in the reaction well 612. The third primer sequences 620 are fixed in the first reaction well 610 and the second reaction well 612; the sequence of the third primer sequence 620 is identical or complementary to the first universal primer sequences 352, 362 or the second universal primer sequences 354, 364 as shown in Fig. 2B. The third primer sequences 620 can be used as primers for a clonal amplification reaction. In the first reaction well 610, the first template molecule 330 is used as a template, and the first amplicon 630 generated is fixed in the first reaction well 610. The first amplicon 630 has a sequence 356' complementary to the first identity sequence tag 356. Correspondingly, in the second reaction well 612, the second template molecule 340 is used as a template, and the second amplicon 640 generated is fixed in the second reaction well 612. The second amplicon 640 has a sequence 366' complementary to the second identity sequence tag 366.

Then, when the thermal cycling reaction is heating up, the first template molecule 330 is separated from the first amplicon 630, and the second template 340 is separated from the second amplicon 640. Then, when the thermal cycling reaction is cooling down, because the reaction solution contains the primer sequences identical or complementary to the first universal primer sequences 352, 362, and primer sequences identical or complementary to the second universal primer sequences 354, 364, a segment identical to the first template molecule 330 is generated by using the first amplicon 630 as a template, and a segment identical to the second template molecules 640 is generated by using the second amplicon 640 as a template. During the subsequent thermal cycles, in the first reaction well 610, the first amplicons 630 fixed in the reaction well are generated through using the third primer sequences 620 fixed in the reaction well as primers, and using the segments identical to the first template 330 as templates. In the second reaction well 612, the second amplicons 640 fixed in the reaction well are generated through using the third primer sequences 620 fixed in the reaction well as primers, and using the segments identical to the first template 640 as templates.

Please refer to Fig. 4D. During the clonal amplification reaction, in the first reaction well 610, many first amplicons 630 fixed in the reaction well are generated and a first amplicon cluster 650 is formed. In the second reaction well 612, many second amplicons 640 fixed in the reaction well are generated and a second amplicon cluster 660 is formed.

According to some embodiments, the amplicon clusters respectively generated from the different target nucleic acid segments (also, different template molecules) are in respective reaction wells, such that the distributions of different amplicon clusters are spatially separated from each other.

In some embodiments, the first amplicons of the first template molecule and the second amplicons of the second template molecule are attached to the inner surfaces of the reaction wells through an intermediate matrix.

According to various embodiments, the amplicon cluster for further analysis is generated based on single-molecule nucleic acid amplification. After amplification, each of the amplicon clusters in the respective reaction wells can have amplicons greater than tens or hundreds of thousands, or millions, depending on the desired assay scale and conditions.

In some embodiments, the amplification reaction of DNA is polymerase chain reaction (PCR), and the substrate is thermally cycled during the amplification reaction.

In some embodiments, alternative nucleic acid amplification techniques can be similarly applied to replace PCR. Such techniques include, for example, isothermal amplification of nucleic acids, ligase chain reaction (LCR), transcription-based amplification systems (TAS), nucleic acid sequence-based amplification (NASBA), strand displacement amplification (SDA), rolling circle amplification (RCA), hyperbranched rolling circle amplification (HRCA), or the like.

In some embodiments, the amplicon may be a double strand DNA molecule or a single strand DNA molecule. As described above, an amplification reaction technique capable of increasing one of the strands can be used.

### D. Identifying the identity sequence tags in the respective reaction wells

In some embodiments, the identity sequence tag in each of the reaction wells is identified by DNA sequencing, polymerase extension, probe hybridization, or probe ligation.

Please refer to Fig. 1. In step 110 of the method 100, the identity codes are identified in the respective reaction wells. If the amplicons from the first target nucleic acid segment or the second target nucleic acid segment are in a reaction well, the first identity sequence tag or the second identity sequence tag will express a positive signal, which means this reaction well has a positive identification of the corresponding template molecule. Therefore, the positive identification indicates the presence of the corresponding encoded target nucleic acid segment in the original sample.

Further sequence-specific analysis can be carried out on these amplicons immobilized on the surface to obtain sequence status. The sequence analysis includes, but is not limited to, labeled probe ligation, single-base extension, DNA sequencing, or melting curve analysis.

In one embodiment, the present disclosure provides a sequential paired-probe ligation method for identifying the identity code of the identity code sequences. In this method, a plurality of probe ligation reaction cycles are performed, and the base compositions of two positions can be simultaneously determined in each individual probe ligation reaction cycle.

In each probe ligation reaction cycle, a pool of labeled identification sequence probes (LISPs) is added. There are four different labels on LISPs with each label representing a designated base (A, C, G, T) at the base interrogation positions.

There are two probe sets in each pool of LISPs, with one set being the 3' labeled identity sequence probe (3' LISPs) that comprise 5' phosphate group and 3' labels, and the other set being the 5' labeled identity sequence probes (5' LISPs) that comprises 5' labels and 3' hydroxy group. All probes required for all possible base combinations at the interrogation positions are included in each pool of LISPs. The base compositions of the interrogation positions can be determined by the fluorescent signals of the ligated pair of LISPs.

In some embodiments, during a probe ligation cycle, two positions in an identity code sequence are interrogated, then the base compositions of the positions are determined depending on the ligated pair of LISPs. In the next probe ligation reaction cycle, another two different positions of the identity code sequence are interrogated successively without repetition.

In some embodiments, labels on the probes are fluorescent labels. A laser is applied to induce fluorescence for carrying out point scanning; a fluorescence imager, or a fluorescent inverted microscope is applied for imaging and then the image results are stored in computers through a CCD image sensor.

In some embodiments, label 1 (L1) is the label associated with A-LISPs at the base interrogation position; label 2 (L2) is the label associated with C-LISPs at the base interrogation position; label 3 (L3) is the label associated with G-LISPs at the base interrogation position; and label 4 (L4) is the label associated with T-LISPs at the base interrogation position.

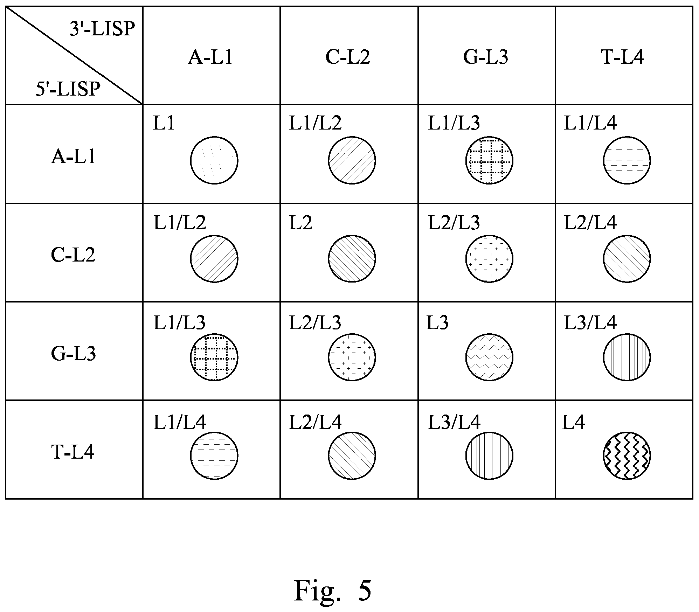

Please refer to Fig. 5, which summarizes the color combinations and corresponding base pair combinations, assuming bases A, C, G, T are labeled with L1, L2, L3 and L4 in both 5'-LISP and 3'-LISP. In the below description, for convenient presentation, the identity code can also be represented by a color code. However, for example, for base combination AC and CA, due to the base combinations of AC and CA generate the same detection signal, the color combination cannot be used to distinguish these two combinations. Therefore, in the coding scheme of the embodiment, there are 10 distinguishable label combinations, including 4 unique combinations of bases, as shown in below Table 1; further, there are 6 equivalent combinations of bases, as shown in below table 2. Thus, each pair of bases in the identity code sequence can provide 10 different identity codes.

Table 1, Unique base code

| Detection signal (Identity code) | L1 | L2 | L3 | L4 |
|---|---|---|---|---|
| Base combination | AA | CC | GG | TT |

Table 2, Equivalent base code

| Detection signal (Identity code) | L1/L2 | L1/L3 | L1/L4 | L2/L3 | L2/L4 | L3/L4 |
|---|---|---|---|---|---|---|
| Base combination 1 | AC | AG | AT | CG | CT | GT |
| Base combination 2 | CA | GA | TA | GC | TC | TG |

If the identity code sequence is 2 bases in length, then the theoretical number of available identity codes is 10. If the identity code sequence is 4 bases in length, then the theoretical number of available identity codes is 10 X 10. If the identity code sequence is 6 bases in length, then the theoretical number of available identity codes is 10 X 10 X 10. Therefore, the identity code sequence is n pair of bases in length, then the theoretical number of available identity codes is $10^n$.

In one embodiment, a number of different tag sequences can be edited, each of the tag sequences has a specific identity code sequence. Therefore, in addition to the first target nucleic acid segment and the second nucleic acid segment, multiple target nucleic acid segments can be detected or quantified simultaneously.

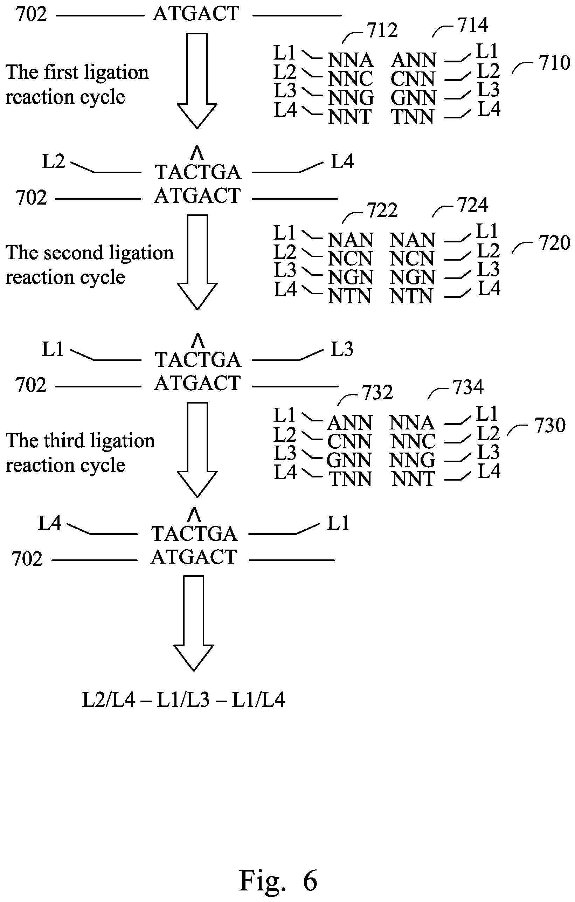

Please refer to Fig. 6, which shows that an identity code of an identity sequence tag is determined by using a method of sequential paired probe-ligation. In some embodiment, two LISPs that are hybridized with the identity code sequence and juxtaposed are ligated by a DNA ligase (e.g., T4 ligase or Tth ligase).

The ligated pair of LISPs probes can remain on the template molecule and the two labels on this pair of LISPs can be detected on the surface of the substrate by a detector. The unligated probes are removed from the surface of the substrate by stringent washing. After a reaction cycle of a pair of probes is ligated, the paired ligated probes are removed from the template, for proceeding to the next ligation cycle. The Tm of the paired ligated probes is significantly different from the Tm of the unligated probe. Removal of the paired ligated probes can be performed by a denaturation operation, such as heating or adding alkaline solutions. The ligation reaction cycle is performed sequentially until all base positions are interrogated.

Please refer to Fig. 6. The first identity sequence tag 256 and the second identity sequence tag 356 respective have identity code sequences. The identity code sequences are identified by probes labeled with fluorescent labels, and the respective identity codes are determined by fluorescence signals. In Fig. 6, for example, the identity code sequence of the identity sequence tag 702 is ATGACT, 6 bases in length; therefore, the identity code of the identity sequence tag 702 can be determined through 3 probe ligation cycles. In each of the ligation reaction, the ligations position of the two probes is in the middle of the identity code sequence (i.e., Between the third base and the fourth base). In the first ligation reaction cycle, the bases to be interrogated are the third base and the fourth base; in the second ligation reaction cycle, the bases to be interrogated are the second base and the fifth base; in the third ligation reaction cycle, the bases to be interrogated are the first base and the sixth base.

In each of the ligation reaction cycles, respective different LISP pools are added, wherein the respective LISPs comprise a base sequence complementary to a part (which is either at the 5' end or 3' end of the identity code sequence) of the identity sequence tag. Each of the 3' end of the 5' LISPs and the 5' end of the 3' LISPs respectively comprises a 3 base sequence, and the N of the 3 base sequence can be A, T, C, or G.

In the first ligation reaction cycle, a LISP pool 710 comprising a 5' LISP set 712 and a 3' LISP set 714 is added. The LISPs of the 5' LISP set 712 are labeled with different fluorescent labels according to the most terminal base of the 3' end (for example, A, C, T, and G are respectively labeled with L1, L2, L3, L4). The LISPs probes of the 3' LISP set 714 are labeled with different fluorescent labels according to the most terminal base of the 5' end (for example, A, C, G, and T are respectively labeled with L1, L2, L3, L4). In the first ligation reaction, the 5' LISP with L2 label and the 3' LISP with L4 label hybridize and bind to the identity sequence tag 702, and then the two probes are ligated. After the unreacted probes are removed, the fluorescent signal (identity code) of L2/L4 can be detected at this time.

After the fluorescent signal is collected, a denaturation operation is performed to remove the ligated paired-probes of the first ligation reaction. Then a second ligation reaction cycle is performed, and a LISP pool 720 comprising 5' LISP set 722 and 3' LISP set 724 is added. Similar to the LISPs of the LISP pool 710, but the LISPs of the 5' LISP set 722 are labeled with different fluorescent labels according to the second base from the 3' end. The LISPs of the 3' LISP set 724 are labeled with different fluorescent labels according to the second base from the 5' end. In the second ligation reaction, the 5' LISP with L1 label and the 3' LISP with L3 label hybridize and bind to the identity sequence tag 702, and then the two probes are ligated. After the unreacted probes are removed, the fluorescent (identity code) of L1/L3 can be detected at this time.

After the fluorescent signals are collected, a denaturation operation is performed to remove the ligated paired-probes of the second ligation reaction. Then a third ligation reaction cycle is performed, and a LISP pool 730 comprising 5' LISP set 732 and 3' LISP set 734 is added. Similar to the LISPs of the LISP pool 710, but the LISPs of the 5' LISP probe set 732 are labeled with different fluorescent labels according to the third base from the 3' end. The LISPs of the 3' LISP set 734 are labeled with different fluorescent labels according to the third base from the 5' end. In the third ligation reaction, the 5' LISP with L4 label and the 3' LISP with L1 label hybridize and bind to the identity sequence tag 702, and then the two probes are ligated. After the unreacted probes are removed, the fluorescent signal (identity code) of L1 /L4 can be detected at this time.

Therefore, the identity code of the identity sequence tag 702 is L2/L4-L1/L3-L1/L4 can be identified by three ligation reaction cycles.

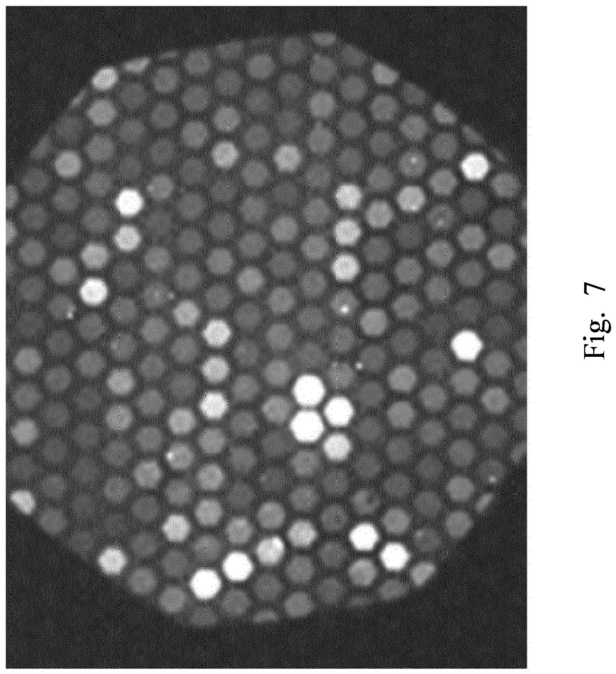

Please refer to Fig. 7, which shows a fluorescent image according to one example. After one of the paired-probe ligation reactions in Fig. 6, the fluorescent image was taken by a fluorescent microscope. Fig. 7 shows the reaction wells with LISPs labeled with fluorescent labels, and the various reaction wells on the same substrate are separated from each other without interference.

In some embodiments, the base interrogation positions are within 5 bases, and preferably within 3 bases, from the ligation point of each LISP, to maintain the base recognition specificity in said paired-probe ligation.

In some embodiments, more than three bases on either side of the ligation site are generally selected by DNA ligase according to the template base sequence. There might be more errors by ligase at base positions away from the ligation site depending on the ligase and ligation conditions. As long as the identification of the identity code sequences in an amplicon cluster (which can have hundreds of thousands of amplicons) is consistent, then some noise can be tolerated.

The length of LISP can vary from 6 to 40 bases, more preferably between 8 to 20 bases. The LISPs can preferably have similar Tm for better hybridization specificity during paired-probe ligation.

In some embodiments, the labels on the LISPs are electrochemical labels or nanoparticles.

E. Quantifying the target sequences

Please refer to Fig. 1. In step 112 of the method 100, a statistical calculation is performed to quantify each of the target nucleic acid segments.

Since each of the template molecules generated from pre-amplification carries a specific identity sequence tag, and the resulting amplicons also carry a sequent generated from the specific identity sequence tag, the readout identity code can be represented as a positive identification of the encoded target nucleic acid segments. Therefore, each target nucleic acid segment can be quantified by counting the number of a specific identity code. Therefore, the total amount of each target molecule (or target nucleic acid segment) in the original sample can be estimated by statistical analysis.

In some embodiments, the method further comprises, the amount of each target nucleic acid segment in the original sample is inferred based on a predetermined dilution factor used in sample preparation. The dilution factor is determined by the number of preamplified template molecules and the volume of the individual reactions, and the dilution factor is adjusted so that each reaction well contains less than one template molecule on average.

In digital PCR reactions, the random distribution of template molecules in a single molecule nucleic acid amplification typically follows Poisson distribution. The random distribution of M template molecules in C reaction chamber follows Poisson distribution. The probability of having at least one template molecule in a given reaction chamber is denoted as p,

$$p = 1 - \left(1 - \frac{1}{C}\right)^M$$

The average concentration of template molecule in each reaction chamber is denoted by $\lambda$:

$$\lambda = MC$$

As the number of reaction chambers becomes arbitrarily large, one can get,

$$\lambda = -\ln(1-p)$$

The probability p can be estimated by counting the positive reaction chambers in digital PCR. Therefore, the average concentration of the template molecule in each reaction well and consequently the amount of the target molecules (i.e., The target nucleic acid segments) in the sample can be statistically calculated.

For 95% confidence interval, the confidence limits of probability p can be expressed as:

$$p \pm 1.96 \sqrt{\frac{p(1-p)}{C}}$$

Since the target template molecules are randomly distributed among these individual reaction wells, some of the reaction wells contain amplicons which are from only one copy of the template molecule (unique positive well), and some contain no amplicon at all (negative wells), some contain amplicons which are from more than one template molecule (complicated positive wells). The location of each template molecule among the individual reaction wells on the substrate may be determined after identifying and decoding the identity codes.

In some embodiments, a statistical calculation is performed to quantify the first target nucleic segment and the second target nucleic segment. The statistical calculation comprises counting the number of reaction wells, the number of positive identifications of the first target nucleic acid segment, and the number of positive identifications of the second target nucleic acid segment.

In some embodiments, each of the target template molecules in the diluted sample solution can be obtained by a calculation comprising counting the number of positive reaction wells, the number of negative reaction wells, and the number of complicated positive reaction wells. Then, each of the corresponding target nucleic acid segments in the original sample can be quantified through the predetermined dilution fold of the sample.

In some embodiments, the sequence composition other than the first identity sequence tag or the second identity sequence tag of the first amplicon or the second amplicon may be further analyzed. For example, a sequence between the two primers in the first amplicon or a sequence between the two primers in the second amplicon can be analyzed.

According to some embodiments, multiple nucleic acid segments can be simultaneously analyzed, the number of the nucleic acid segments may be, for example, 5, 10, 50, 100, 200, 300, 400, 500, 600, 700, 800, 1000, 2000, 3000, 4000, 5000, 6000, 7000, or 8000.

According to one embodiment, the method further comprises using certain target segments (for example, segments of housekeeping genes) that have known numbers of copies in each genome of interest, as quantification reference segments in the analysis. In some embodiments, after the pre-amplification reaction, each of the template molecules generated from a reference nucleic acid segment has an embedded identity sequence tag.

F. The embodiments of the present disclosure provide a flow cell and a system for multiplex nucleic acid analysis

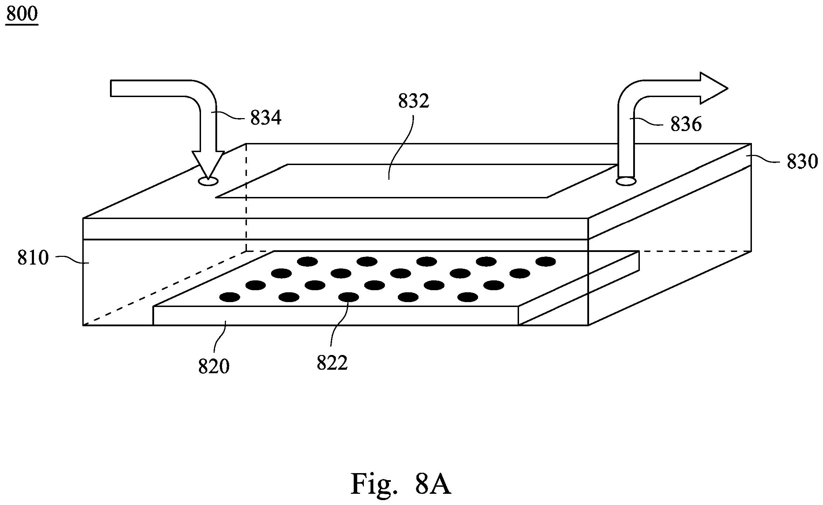

Please refer to Fig. 8A, which shows a schematic diagram of a flow cell according to some embodiments of the present disclosure. The flow cell 800 comprises an outer casing 810. A substrate 820 is within the flow cell 800. A plurality of reaction wells 822 are on the substrate 820. The upper cover 830 having an optically transparent window is over the outer casing 810. The upper cover 830 has a liquid inlet 834 and a liquid outlet 836 in the opposite configuration.

According to some embodiments, during the detection process, a suitably diluted sample solution having template molecules is introduced through the liquid inlet 834, such that the sample solution flows along the surface of substrate 820 and is uniformly filled in the respective reaction wells 822. Then, the excess sample solution on the surface of the substrate is then removed through the liquid outlet 836. The template molecules in the sample solution are thus randomly distributed into each reaction wells 822.

Then, a liquid immiscible with water is introduced through the liquid inlet 834 to fully cover each of the reaction wells 822 for isolating the respective reaction wells and preventing water evaporation.

According to one embodiment, the upper cover is optically transparent to directly detect image signals of the respective reaction wells on the substrate.

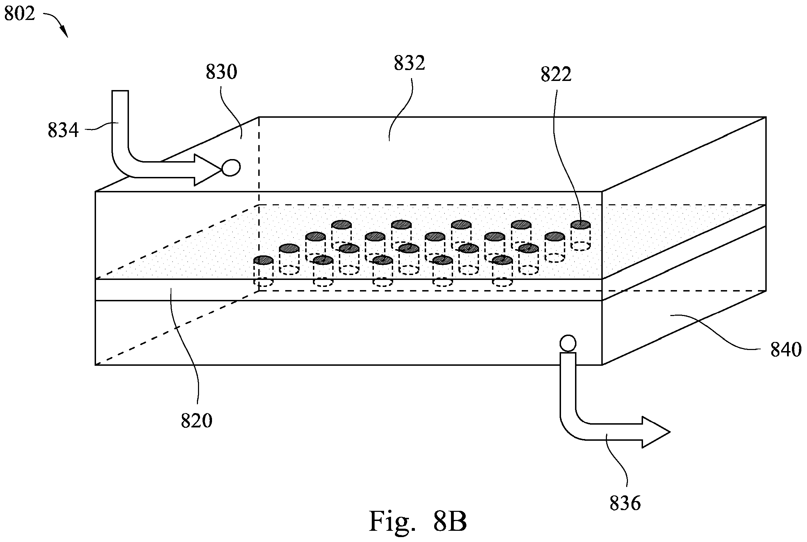

Please refer to Fig. 8B, which illustrates a schematic diagram of a flow cell according to some embodiments. The chamber 802 has a substrate 820 having a plurality of through-hole reaction wells 822. The upper cover 830 has a liquid inlet 834, and the lower cover 840 has a liquid outlet 836 in an opposite configuration. The upper cover 830 has an optical detection area 832.

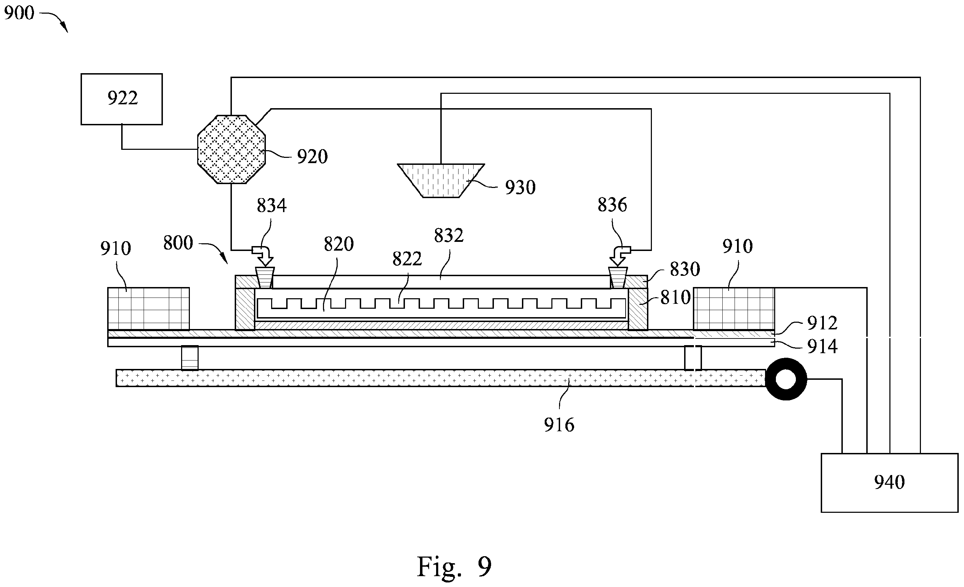

Fig. 9 illustrates a system 900 for multiplex nucleic acid analysis. In the system 900, the flow cell 800 is disposed on a heat conducting layer 912, which are attached to one or two (optional) thermoelectric heating and cooling units 910 for regulating the temperature of the surface of the substrate of the flow cell. The thermal isolation layer 914 is optionally disposed under the heat conducting layer 912. The entire assembly is mounted on an x-y precision moving stage 916. The scanning area of the detection window in the flow cell can be adjusted through the moving stage 916. A fluidic system 920 is connected to a reagent unit 922, where all necessary reagents for the assay are stored and optionally kept at a specified temperature. The fluidic system controls the delivery and removal of reagents from the inlet and outlet of the flow cell, as well as waste control. A detection unit 930 is mounted directly facing a detection window 832 of the flow cell 800, and the detection unit 930 is capable of automatically maintaining the focus and detecting all the optical labels used in the assay. All of the control and data processing are handled by a computing unit 940.

According to an embodiment, the heat conducting layer is made of a good thermal conductor, such as aluminum, copper, or a combination thereof.

According to an embodiment, the detection unit comprises a fluorescence microscope having filter cubes for different excitation and emission spectra. The detection unit comprises a CCD imaging camera.

In some embodiments, more than one flow cell can be mounted on the system described above to optimize the efficiency of the system and provide flexibility in sample analysis. In this system, different flow cells can be programmed to run different assays. For example, sequential probe ligation reactions can be run in one of the flow cells, while fluorescence imaging can be processed in another flow cell of the system. More than one fluidic control unit or detection unit can be included as well.

The advantages of the present disclosure

In the prior art, using emulsion droplet and fixing the amplicons on magnetic beads to perform digital PCR reaction may cause aggregation of some magnetic beads due to the magnetic field. Aggregation of microparticles is undesirable and could pose difficulties in the imaging process. Embodiments of the present disclosure provide methods for improving image accuracy and accuracy. Through the physically isolated reaction wells, the respective template molecules are amplified, and the respective identity codes are identified *in situ* respectively in the reaction wells.

The respective reaction wells are configured to be physically isolated from each other on the substrate; therefore, the detection process can be better regulated, and the sample volume can be better quantified, so that the results of the respective PCR reactions are more reliable.

In some embodiments of the present disclosure, since the position and the parameters of the respective reaction wells on the substrate are consistent or predetermined. Further, the size, number, and volume of the suitable reaction wells can be adjusted according to the detection requirements, for example, the volumes of various reaction wells can be the same or different. Therefore, the method of the embodiments facilitates automated detection of multiplex nucleic acid segments.

In the prior art, for performing digital PCR with emulsion droplets, the microbeads or magnetic beads with primers are used for clonal amplification. Hence, in the step of sample solution distribution, some of the reaction sites may have no distribution of the bead or magnetic bead and then result in false negative results. In contrast, another advantage of the embodiments of the present disclosure is that the amplicon is immobilized in the reaction well by the primer immobilized in the reaction well, so that there are moderate or sufficient primers present in each of the reaction wells during clonal amplification.

Another advantageous effect of the present disclosure is that during identification of the identity code sequence, a pre-decoding step for determining the positions of the identity code sequences is not required; the encoded corresponding target nucleic acid segment can be directly determined by the signals from the identity code sequence.

Claims

1. A method for analysing nucleic acids in a sample, the nucleic acids containing a first target nucleic acid segment (212) and a second target nucleic acid segment (222), the method comprising:

(a) performing a pre-amplification to generate first template molecules (230) from the first target nucleic acid segment (212) and generate second template molecules (240) from the second target nucleic acid segment (222), wherein each of the first template molecules (230) comprises sequences generated from a first universal primer sequence (252), a second universal sequence (272), and a first identity sequence tag (256), and each of the second template molecules (240) comprises sequences generated from the first universal primer sequence (262), the second universal sequence (282), and a second identity sequence tag (266);

(b) dispersing the sample to a substrate (820) in a flow cell (800) for random distribution of the first template molecules (230) and the second template molecules (240) among respective reaction wells (822) of the substrate (820), wherein the number of the first template molecules (230) and the second template molecules (240) in each of the reaction wells (822) is less than 1 on average;

(c) *in situ* performing nucleic acid amplification reaction to generate a plurality of first amplicons (530) from the first template molecules (230) and a plurality of second amplicons (540) from the second template molecules (240), wherein the first amplicons (530) and the second amplicons (540) are fixed on respective inner surfaces within the respective reaction wells (822) of the substrate (820) in the flow cell (800);

(d) *in situ* identifying the first identity sequence tag (256) of the first template molecules (230) of the first amplicons (530) and the second identity tag (266) of the second template molecules (240) of the second amplicons (540) on the respective inner surfaces within the respective reaction wells (822) of the substrate (820) in the flow cell (800), wherein a signal of the first identity sequence tag (256) represents a positive identification of the first target nucleic acid segment (212), and a signal of the second identity sequence tag (266) represents a positive identification of the second target nucleic acid segment (222); and

(e) performing statistical calculation for quantifying the first target nucleic acid segment (212) and the second target nucleic acid segment (222) through the number of the reaction wells (822), the number of the positive identification of the first target nucleic acid segment (212), and the number of the positive identification of the second target nucleic acid segments (222).

2. The method of claim 1, wherein the first identity sequence tag (256) comprises a first identity code sequence, and the second identity sequence tag (266) comprises a second identity code sequence.

3. The method of claim 1 or 2, wherein the pre-amplification reaction comprises a gene-specific amplification reaction; or

   wherein the pre-amplification reaction comprises a gene-specific 5' exonuclease cleavage reaction; or
   wherein the pre-amplification reaction comprises a linkage reaction of target-specific probes and then a universal primer sequence based amplification reaction, and the target-specific probes comprise the first identity sequence tag or the second identity sequence tag.

4. The method of any one of claims 1 to 3, wherein the sample further comprises a reference nucleic acid segment, and each of third template molecules generated from the reference nucleic acid segment has a third identity sequence.

5. The method of any one of claims 1 to 4, wherein the first template molecules (230) further comprise a sequence generated from the first target nucleic acid segment (232), and the second template molecules (240) further comprise a sequence generated from the second target nucleic acid segment (242).

6. The method of any one of claims 1 to 5, wherein the reaction wells (822) of the substrate (820) are recessed spaces formed on a surface of the substrate (820); and/or

   wherein the reaction wells (450) of the substrate (460) are through holes formed through the substrate (460); and/or
   wherein the reaction wells (412) have the same volume; and/or
   wherein the reaction wells are circular or polygonal in shape; and/or
   wherein the reaction wells of the substrate (420) have different shapes.

7. The method of any one of claims 1 to 6, further comprising: after step (a), quantifying nucleic acid amount of the first template molecules (230) or the second template molecules (240), and determining a dilution factor of the sample.

8. The method of any one of claims 1 to 7, further comprising: before step (c), adding a water-immiscible liquid to the substrate (820) to cover and isolate the reaction wells (822) from each other.

9. The method of any one of claims 1 to 8, wherein third primer sequences (520) are fixed in the reaction wells, the third primer sequences comprise sequences complementary or identical to the first universal primer sequence (252) or the second universal primer sequence (272).

10. The method of claim 9, wherein the first amplicons (530) and the second amplicons (540) are attached to inner surfaces of the reaction wells (510) through the third primer sequences (520).

11. The method of any one of claims 1 to 10, wherein the first amplicons (530) generated from the first template molecules (230) and the second amplicons (540) generated from the second template molecules (240) are immobilized on inner surfaces of the reaction wells (822) by an intermediate matrix.

12. The method of any one of claims 1 to 11, wherein the step of (d) is performed by DNA sequencing, polymerase extension, probe hybridization, or probe ligation.

13. The method of any one of claims 1 to 12, after the step (d), further comprises analyzing a sequence composition other than the first identity sequence tag of the first amplicons (530), or a sequence composition other than the second identity sequence tag of the second amplicons (540).

14. The method of any one of claims 1 to 13, wherein the first target nucleic acid segment (212) and the second target nucleic acid segment (222) are a same nucleic acid segment; or
wherein the first target nucleic acid segment (212) and the second target nucleic acid segment (222) are different nucleic acid segments.

15. The method of any one of claims 1 to 14, wherein step (b) further comprises a reaction solution flowing over the respective inner surfaces of the reaction wells (822) of the substrate (820).

**Patentansprüche**

1. Verfahren zur Analyse von Nukleinsäuren in einer Probe, wobei die Nukleinsäuren ein erstes Ziel-Nukleinsäuresegment (212) und ein zweites Ziel-Nukleinsäuresegment (222) enthalten, wobei das Verfahren umfasst:

(a) Durchführen einer Voramplifikation, um erste Matrizenmoleküle (230) aus dem ersten Ziel-Nukleinsäuresegment (212) zu erzeugen und zweite Matrizenmoleküle (240) aus dem zweiten Ziel-Nukleinsäuresegment (222) zu erzeugen, wobei jedes der ersten Matrizenmoleküle (230) Sequenzen umfasst, die aus einer ersten universellen Primersequenz (252), einer zweiten universellen Sequenz (272) und einem ersten Identitätssequenz-Tag (256) erzeugt wurden, und jedes der zweiten Matrizenmoleküle (240) Sequenzen umfasst, die aus der ersten universellen Primersequenz (262), der zweiten universellen Sequenz (282) und einem zweiten Identitätssequenz-Tag (266) erzeugt wurden;
(b) Auftragen der Probe auf einem Substrat (820) in einer Durchflusszelle (800) zur zufälligen Verteilung der ersten Matrizenmoleküle (230) und der zweiten Matrizenmoleküle (240) auf entsprechende Reaktionsvertiefungen (822) des Substrats (820), wobei die Anzahl der ersten Matrizenmoleküle (230) und der zweiten Matrizenmoleküle (240) in jeder der Reaktionsvertiefungen (822) im Durchschnitt weniger als 1 beträgt;
(c) *in situ* Durchführen einer Nukleinsäure-Amplifikationsreaktion, um eine Vielzahl von ersten Amplicons (530) aus den ersten Matrizenmolekülen (230) und eine Vielzahl von zweiten Amplicons (540) aus den zweiten Matrizenmolekülen (240) zu erzeugen, wobei die ersten Amplicons (530) und die zweiten Amplicons (540) auf entsprechenden inneren Oberflächen innerhalb der jeweiligen Reaktionsvertiefungen (822) des Substrats (820) in der Durchflusszelle (800) fixiert werden;
(d) *in situ* Identifizierung des ersten Identitätssequenz-Tags (256) der ersten Matrizenmoleküle (230) der ersten Amplicons (530) und des zweiten Identitäts-Tags (266) der zweiten Matrizenmoleküle (240) der zweiten Amplicons (540) auf den entsprechenden inneren Oberflächen innerhalb der jeweiligen Reaktionsvertiefungen (822) des Substrats (820) in der Durchflusszelle (800), wobei ein Signal des ersten Identitätssequenz-Tags (256) eine positive Identifizierung des ersten Ziel-Nukleinsäuresegments (212) darstellt, und ein Signal des zweiten Identitätssequenz-Tags (266) eine positive Identifizierung des zweiten Ziel-Nukleinsäuresegments (222) darstellt; und
(e) Durchführen einer statistischen Berechnung zur Quantifizierung des ersten Ziel-Nukleinsäuresegments (212) und des zweiten Ziel-Nukleinsäuresegments (222) über die Anzahl der Reaktionsvertiefungen (822), die Anzahl der positiven Identifizierungen des ersten Ziel-Nukleinsäuresegments (212) und die Anzahl der positiven Identifizierungen des zweiten Ziel-Nukleinsäuresegments (222).

2. Verfahren nach Anspruch 1, wobei das erste Identitätssequenz-Tag (256) eine erste Identitätscodesequenz umfasst und das zweite Identitätssequenz-Tag (266) eine zweite Identitätscodesequenz umfasst.

3. Verfahren nach Anspruch 1 oder 2, wobei die Voramplifikationsreaktion eine genspezifische Amplifikationsreaktion umfasst; oder

wobei die Voramplifikationsreaktion eine genspezifische 5'-Exonuclease-Spaltungsreaktion umfasst; oder wobei die Voramplifikationsreaktion eine Verknüpfungsreaktion von zielspezifischen Sonden und dann eine auf einer universellen Primersequenz basierende Amplifikationsreaktion umfasst, und die zielspezifischen Sonden das erste Identitätssequenz-Tag oder das zweite Identitätssequenz-Tag umfassen.

4. Verfahren nach einem der Ansprüche 1 bis 3, wobei die Probe ferner ein Referenz-Nukleinsäuresegment umfasst und jedes der aus dem Referenz-Nukleinsäuresegment erzeugten dritten Matrizenmoleküle eine dritte Identitätssequenz aufweist.

5. Verfahren nach einem der Ansprüche 1 bis 4, wobei die ersten Matrizenmoleküle (230) ferner eine aus dem ersten Ziel-Nukleinsäuresegment (232) erzeugte Sequenz umfassen und die zweiten Matrizenmoleküle (240) ferner eine aus dem zweiten Ziel-Nukleinsäuresegment (242) erzeugte Sequenz umfassen.

6. Verfahren nach einem der Ansprüche 1 bis 5, wobei die Reaktionsvertiefungen (822) des Substrats (820) vertiefte Räume sind, die auf einer Oberfläche des Substrats (820) ausgebildet sind; und/oder

   wobei die Reaktionsvertiefungen (450) des Substrats (460) Durchgangslöcher sind, die durch das Substrat (460) hindurch ausgebildet sind; und/oder
   wobei die Reaktionsvertiefungen (412) das gleiche Volumen haben; und/oder
   wobei die Reaktionsvertiefungen eine kreisförmige oder polygonale Form haben; und/oder
   wobei die Reaktionsvertiefungen des Substrats (420) unterschiedliche Formen haben.

7. Verfahren nach einem der Ansprüche 1 bis 6, ferner umfassend: nach Schritt (a) die Quantifizierung der Nukleinsäuremenge der ersten Matrizenmoleküle (230) oder der zweiten Matrizenmoleküle (240) und Bestimmung eines Verdünnungsfaktors der Probe.

8. Verfahren nach einem der Ansprüche 1 bis 7, ferner umfassend: vor Schritt (c) die Zugabe einer mit Wasser nicht mischbaren Flüssigkeit zu dem Substrat (820), um die Reaktionsvertiefungen (822) zu bedecken und voneinander zu isolieren.

9. Verfahren nach einem der Ansprüche 1 bis 8, wobei dritte Primersequenzen (520) in den Reaktionsvertiefungen fixiert werden, wobei die dritten Primersequenzen Sequenzen umfassen, die komplementär oder identisch zu der ersten universellen Primersequenz (252) oder der zweiten universellen Primersequenz (272) sind.

10. Verfahren nach Anspruch 9, wobei die ersten Amplicons (530) und die zweiten Amplicons (540) durch die dritten Primersequenzen (520) an innere Oberflächen der Reaktionsvertiefungen (510) gebunden werden.

11. Verfahren nach einem der Ansprüche 1 bis 10, wobei die ersten Amplicons (530), die aus den ersten Matrizenmolekülen (230) erzeugt werden, und die zweiten Amplicons (540), die aus den zweiten Matrizenmolekülen (240) erzeugt werden, durch eine Zwischenmatrix auf inneren Oberflächen der Reaktionsvertiefungen (822) immobilisiert werden.

12. Verfahren nach einem der Ansprüche 1 bis 11, wobei der Schritt (d) durch DNA-Sequenzierung, Polymeraseverlängerung, Sondenhybridisierung oder Sondenligierung durchgeführt wird.

13. Verfahren nach einem der Ansprüche 1 bis 12, welches nach dem Schritt (d) ferner das Analysieren einer anderen Sequenzzusammensetzung als des ersten Identitätssequenz-Tags der ersten Amplicons (530) oder einer anderen Sequenzzusammensetzung als des zweiten Identitätssequenz-Tags der zweiten Amplicons (540) umfasst.

14. Verfahren nach einem der Ansprüche 1 bis 13, wobei das erste Ziel-Nukleinsäuresegment (212) und das zweite Ziel-Nukleinsäuresegment (222) das gleiche Nukleinsäuresegment sind; oder wobei das erste Ziel-Nukleinsäuresegment (212) und das zweite Ziel-Nukleinsäuresegment (222) unterschiedliche Nukleinsäuresegmente sind.

15. Verfahren nach einem der Ansprüche 1 bis 14, wobei Schritt (b) ferner umfasst, dass eine Reaktionslösung über die jeweiligen inneren Oberflächen der Reaktionsvertiefungen (822) des Substrats (820) fließt.

**Revendications**

1. Procédé d'analyse d'acides nucléiques dans un échantillon, les acides nucléiques contenant un premier segment d'acide nucléique cible (212) et un deuxième segment d'acide nucléique cible (222), le procédé comprenant les étapes consistant à :

   (a) réaliser une pré-amplification pour générer des premières molécules matrices (230) à partir du premier segment d'acide nucléique cible (212) et générer des deuxièmes molécules matrices (240) à partir du deuxième segment d'acide nucléique cible (222), où chacune des premières molécules matrices (230) comprend des séquences générées à partir d'une première séquence d'amorce universelle (252), d'une deuxième séquence universelle (272) et d'une première étiquette de séquence d'identité (256), et chacune des deuxièmes molécules matrices (240) comprend des séquences générées à partir de la première séquence d'amorce universelle (262), de la deuxième séquence universelle (282) et d'une deuxième étiquette de séquence d'identité (266) ;
   (b) disperser l'échantillon sur un substrat (820) dans une cellule d'écoulement (800) pour une distribution aléatoire des premières molécules matrices (230) et des deuxièmes molécules matrices (240) entre des puits de réaction respectifs (822) du substrat (820), où le nombre des premières molécules matrices (230) et des deuxièmes molécules matrices (240) dans chacun des puits de réaction (822) est inférieur à 1 en moyenne ;
   (c) réaliser *in situ* une réaction d'amplification d'acide nucléique pour générer une pluralité de premiers amplicons (530) à partir des premières molécules matrices (230) et une pluralité de deuxièmes amplicons (540) à partir des deuxièmes molécules matrices (240), où les premiers amplicons (530) et les deuxièmes amplicons (540) sont fixés sur des surfaces internes respectives dans les puits de réaction respectifs (822) du substrat (820) dans la cellule d'écoulement (800) ;
   (d) identifier *in situ* la première étiquette de séquence d'identité (256) des premières molécules matrices (230) des premiers amplicons (530) et la deuxième étiquette d'identité (266) des deuxièmes molécules matrices (240) des deuxièmes amplicons (540) sur les surfaces internes respectives dans les puits de réaction respectifs (822) du substrat (820) dans la cellule d'écoulement (800), où un signal de la première étiquette de séquence d'identité (256) représente une identification positive du premier segment d'acide nucléique cible (212), et un signal de la deuxième étiquette de séquence d'identité (266) représente une identification positive du deuxième segment d'acide nucléique cible (222) ; et
   (e) réaliser un calcul statistique pour quantifier le premier segment d'acide nucléique cible (212) et le deuxième segment d'acide nucléique cible (222) par le nombre des puits de réaction (822), le nombre de l'identification positive du premier segment d'acide nucléique cible (212), et le nombre de l'identification positive des deuxièmes segments d'acide nucléique cibles (222) .

2. Procédé de la revendication 1, dans lequel la première étiquette de séquence d'identité (256) comprend une première séquence de code d'identité, et la deuxième étiquette de séquence d'identité (266) comprend une deuxième séquence de code d'identité.

3. Procédé de la revendication 1 ou 2, dans lequel la réaction de pré-amplification comprend une réaction d'amplification spécifique à un gène ; ou

   dans lequel la réaction de pré-amplification comprend une réaction de clivage par exonucléase 5' spécifique à un gène ; ou
   dans lequel la réaction de pré-amplification comprend une réaction de liaison de sondes spécifiques à une cible et ensuite une réaction d'amplification basée sur une séquence d'amorce universelle, et les sondes spécifiques à une cible comprennent la première étiquette de séquence d'identité ou la deuxième étiquette de séquence d'identité.

4. Procédé de l'une quelconque des revendications 1 à 3, dans lequel l'échantillon comprend en outre un segment d'acide nucléique de référence, et chacune de troisièmes molécules matrices générées à partir du segment d'acide nucléique de référence a une troisième séquence d'identité.

5. Procédé de l'une quelconque des revendications 1 à 4, dans lequel les premières molécules matrices (230) comprennent en outre une séquence générée à partir du premier segment d'acide nucléique cible (232), et les deuxièmes molécules matrices (240) comprennent en outre une séquence générée à partir du deuxième segment d'acide nucléique cible (242).

6. Procédé de l'une quelconque des revendications 1 à 5, dans lequel les puits de réaction (822) du substrat (820) sont des espaces en évidement formés sur une surface du substrat (820) ; et/ou

dans lequel les puits de réaction (450) du substrat (460) sont des trous traversants formés à travers le substrat (460) ; et/ou

dans lequel les puits de réaction (412) ont le même volume ; et/ou

dans lequel les puits de réaction sont de forme circulaire ou polygonale ; et/ou

dans lequel les puits de réaction du substrat (420) ont des formes différentes.

7. Procédé de l'une quelconque des revendications 1 à 6, comprenant en outre les étapes consistant à : après l'étape (a), quantifier la quantité d'acide nucléique des premières molécules matrices (230) ou des deuxièmes molécules matrices (240), et déterminer un facteur de dilution de l'échantillon.

8. Procédé de l'une quelconque des revendications 1 à 7, comprenant en outre l'étape consistant à : avant l'étape (c), ajouter un liquide non miscible à l'eau au substrat (820) pour recouvrir et isoler les puits de réaction (822) les uns des autres.

9. Procédé de l'une quelconque des revendications 1 à 8, dans lequel des troisièmes séquences d'amorce (520) sont fixées dans les puits de réaction, les troisièmes séquences d'amorce comprennent des séquences complémentaires ou identiques à la première séquence d'amorce universelle (252) ou à la deuxième séquence d'amorce universelle (272).

10. Procédé de la revendication 9, dans lequel les premiers amplicons (530) et les deuxièmes amplicons (540) sont attachés à des surfaces internes des puits de réaction (510) à travers les troisièmes séquences d'amorce (520).

11. Procédé de l'une quelconque des revendications 1 à 10, dans lequel les premiers amplicons (530) générés à partir des premières molécules matrices (230) et les deuxièmes amplicons (540) générés à partir des deuxièmes molécules matrices (240) sont immobilisés sur des surfaces internes des puits de réaction (822) par une matrice intermédiaire.

12. Procédé de l'une quelconque des revendications 1 à 11, dans lequel l'étape de (d) est réalisée par séquençage d'ADN, extension par polymérase, hybridation de sonde ou ligature de sonde.

13. Procédé de l'une quelconque des revendications 1 à 12, après l'étape (d), comprend en outre l'étape consistant à analyser une composition de séquence autre que la première étiquette de séquence d'identité des premiers amplicons (530), ou une composition de séquence autre que la deuxième étiquette de séquence d'identité des deuxièmes amplicons (540).

14. Procédé de l'une quelconque des revendications 1 à 13, dans lequel le premier segment d'acide nucléique cible (212) et le deuxième segment d'acide nucléique cible (222) représentent un même segment d'acide nucléique ; ou dans lequel le premier segment d'acide nucléique cible (212) et le deuxième segment d'acide nucléique cible (222) sont des segments d'acide nucléique différents.

15. Procédé de l'une quelconque des revendications 1 à 14, dans lequel l'étape (b) comprend en outre une solution réactionnelle s'écoulant sur les surfaces internes respectives des puits de réaction (822) du substrat (820).

100 —

Receiving a nucleic acid sample containing target nucleic acid segments — 102

Performing pre-amplification reaction to amplify the target nucleic acid segments and insert an identity sequence tag comprising an identity code sequence in each of the template molecules generated from the pre-amplification — 104

Dispersing the sample to a substrate for random distribution of the template molecules among various reaction wells of the substrate, wherein the number of the template molecules in each of the reaction wells is less than 1 on average — 106

Performing nucleic acid amplification reaction for the template molecules in each of the reaction wells, the resulting amplicons are attached to the respective reaction wells through the primers fixed in the reaction wells — 108

Identifying the identity code sequence in the respective reaction wells — 110

Performing statistical calculation to quantify each of the target nucleic acid segments — 112

Fig. 1

Fig. 2A

EP 3 453 769 B1

Fig. 2B

EP 3 453 769 B1

Fig. 3A

Fig. 3B

Fig. 3C

EP 3 453 769 B1

Fig. 3D

EP 3 453 769 B1

Fig. 4A

EP 3 453 769 B1

Fig. 4B

EP 3 453 769 B1

Fig. 4C

Fig. 4D

EP 3 453 769 B1

| 5'-LISP \ 3'-LISP | A-L1 | C-L2 | G-L3 | T-L4 |
|---|---|---|---|---|
| A-L1 | L1 | L1/L2 | L1/L3 | L1/L4 |
| C-L2 | L1/L2 | L2 | L2/L3 | L2/L4 |
| G-L3 | L1/L3 | L2/L3 | L3 | L3/L4 |
| T-L4 | L1/L4 | L2/L4 | L3/L4 | L4 |

Fig. 5

702 ——————— ATGACT ———————

The first ligation reaction cycle

L1 ⟍ NNA   ANN ⟋ L1   ⌐ 710
L2 ⟍ NNC   CNN ⟋ L2
L3 ⟍ NNG   GNN ⟋ L3
L4 ⟍ NNT   TNN ⟋ L4

⌐712    ⌐714

L2 ⟍ ——————— TACTGA ——————— ⟋ L4
702 ——————— ATGACT ———————

The second ligation reaction cycle

L1 ⟍ NAN   NAN ⟋ L1   ⌐ 720
L2 ⟍ NCN   NCN ⟋ L2
L3 ⟍ NGN   NGN ⟋ L3
L4 ⟍ NTN   NTN ⟋ L4

⌐722    ⌐724

L1 ⟍ ——————— TACTGA ——————— ⟋ L3
702 ——————— ATGACT ———————

The third ligation reaction cycle

L1 ⟍ ANN   NNA ⟋ L1   ⌐ 730
L2 ⟍ CNN   NNC ⟋ L2
L3 ⟍ GNN   NNG ⟋ L3
L4 ⟍ TNN   NNT ⟋ L4

⌐732    ⌐734

L4 ⟍ ——————— TACTGA ——————— ⟋ L1
702 ——————— ATGACT ———————

L2/L4 – L1/L3 – L1/L4

# Fig. 6

Fig. 7

800

Fig. 8A

Fig. 8B

EP 3 453 769 B1

Fig. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010115100 A1

**Non-patent literature cited in the description**

- *Castiglionic Appl. Environ. Microbiol.,* 2004, vol. 70 (12), 7161